# EUROPEAN PATENT APPLICATION

(11) **EP 4 825 984 A1**
(43) Date of publication of application: **21.10.2026**
(21) Application number: 25171318.6
(22) Date of filing: 17.04.2025
(51) Int. Cl.: C12Q 1/6895, C12Q 1/70

(54) **IN-FIELD DETECTION OF PLANT PATHOGENS**

(71) Applicant: KWS SAAT SE & Co. KGaA, 37574 Einbeck (DE)
(72) Inventor: Purahong, Witoon, 37574 Einbeck (DE)
(74) Representative: Eisenführ Speiser

(57) **Abstract**

The invention relates to an *in situ* loop mediated isothermal amplification method for identifying at least one nucleic acid sequence in at least one plant material within short time period. Further, the invention relates to transmitting the result of said method to a database via a wireless mobile device and integrating said result with field-mapping data. The invention further relates to a simplified method of sample collection from a plant in an agricultural field. The invention also relates to a system, preferably a portable system, for obtaining at least one test result and optionally allowing the transmittance of a test result via a wireless mobile device to a database. Further, the invention relates to a primer set for detecting the presence or absence of a Beta spp. specific pathogen. The invention also relates to the use of said system for performing said *in situ* loop mediated isothermal amplification method.

## Description

### Technical Field

The invention relates to an *in situ* loop mediated isothermal amplification method for identifying at least one nucleic acid sequence in at least one plant material within short time period. Further, the invention relates to transmitting the result of said method to a database via a wireless mobile device and integrating said result with field-mapping data. The invention further relates to a simplified method of sample collection from a plant in an agricultural field. The invention also relates to a system, preferably a portable system, for obtaining at least one test result and optionally allowing the transmittance of a test result via a wireless mobile device to a database. Further, the invention relates to a primer set for detecting the presence or absence of a Beta spp. specific pathogen. The invention also relates to the use of said system for performing said *in situ* loop mediated isothermal amplification method.

### Background of the Invention

PCR-based methods remain a cornerstone of plant pathogen detection due to their high specificity and sensitivity. By targeting pathogen-specific DNA sequences, PCR enables both qualitative and quantitative diagnostics. However, these assays demand high-purity DNA, free from inhibitors like polysaccharides and phenolic compounds, which can interfere with amplification reactions (Lopez et al., 2009). DNA extraction methods vary widely in complexity and yield, impacting consistency and reproducibility. Though PCR offers a manifold greater sensitivity than serological methods, its dependence on thermal cycling, expensive equipment, and technical expertise limits its accessibility and suitability for field-based applications (Khater et al., 2017).

In contrast, isothermal amplification methods are gaining momentum as a more practical alternative, particularly for *in situ* and high-throughput diagnostics. Unlike PCR, these techniques amplify nucleic acids at a constant temperature, eliminating the need for a thermal cycler. Isothermal approaches have demonstrated advantages in terms of sensitivity and selectivity (Oliveira et al., 2021). Among these, loop-mediated isothermal amplification (LAMP), has emerged as a suitable method for phytopathogen detection (Notomi et al., 2000; Harmon et al., 2003).

LAMP makes use of a stand-displacement polymerase and a primer set that recognizes six distinct regions on the target DNA. It can amplify trace amounts of DNA to femtogram levels within an hour under isothermal conditions - typically around 65 °C. Because it operates without thermal cycling, LAMP can be performed with simple devices like a water bath or heat block. However, one of the main challenges with LAMP is its primer design complexity. Unlike conventional PCR, which typically uses just two primers, LAMP requires a carefully designed set of at least four to six primers that recognize multiple regions of the target sequence. This intricate primer system makes assay development more challenging and requires extensive optimization. Designing effective primers can be particularly difficult for organisms with high genetic variability, as mutations in the target regions may disrupt primer binding and lead to inconsistent results.

In addition, commercial LAMP assays, such as "Loopamp^{®}" (Eiken chemical Co., Ltd , Japan) or "AmplifyRP^{®} Acceler8^{®} for BBTV" (Agdia Inc., U.S.) entail multiple pipetting steps and the careful combination of separate reagents, each of which must be precisely measured and stored under strict conditions to maintain their activity. Users may need to operate specialized instruments for the detection of amplified products, such as fluorescence readers, and interpret complex readouts that demand substantial technical expertise. In many cases, environmental controls are also necessary to prevent contamination and ensure reliability, resulting in the need for a well-equipped laboratory space. These factors collectively make existing LAMP protocols laborious to implement, by limiting their accessibility to highly trained personnel, and present challenges for *in situ* or resource-limited testing scenarios.

While LAMP's assays have been conducted with plant specimen, its use in sugar beet - a crop of high economic importance - remains severely underexplored.

One of the few exceptions is the work by Shrestha et al., who successfully applied LAMP for the detection of *Cercospora beticola* in sugar beet leaves. Their findings indicate that LAMP may achieve both specificity and sensitivity in identifying *C. beticola* infections. Yet, despite this promising outcome, several practical limitations were evident in their methodology. The protocol requires lengthy incubation periods and relied on samples that had been stored at - 80°C, which is impractical for rapid field diagnostics. Moreover, the process involves laborious multiple pipetting steps, necessitating trained personnel and access to lab-grade equipment - factors that severely limit its scalability and *in situ* usability. Crucially, the study also lacks a user-friendly, deployable read-out format.

Another study by Pozharskiy et al., 2024, targeted beet necrotic yellow vein virus using a LAMP assay. While successful in detection, their methodology was heavily reliant on traditional RNA extraction kits and reverse transcription steps. These requirements not only demand a controlled lab environment but also introduce logistical and cost-related barriers that are incompatible with rapid, low-resource settings, not addressing the need of streamlined, field-deployable diagnostics.

Beyond the scarcity of sugar beet-focused assays, a so far unresolved issue inherent to LAMP as a method is its pronounced vulnerability to contamination, as described in US11345970**.** The high efficiency of LAMP amplification, while advantageous for sensitivity, becomes a liability *in situ,* as it exacerbates the risk of cross-contamination and false-positive results. This concern is consistently reflected in the literature, such as by Gomez Gutierrez & Goodwin, 2022 and Muthukumar et al. 2024, who both highlight the susceptibility of LAMP assays to contamination from environmental aerosols and previous reactions. This systemic issue presents a serious bottleneck for reliable diagnostics, especially when assays are performed *in situ,* i.e. outside of controlled laboratory conditions. Especially when assays are conducted in natural environments, cross-contamination from neighbouring plants becomes a significant compounding factor. This risk is particularly acute in dense agricultural settings, where physical proximity facilitates the passive transfer of microscopic plant material, including pollen, leaf exudates, and surface residues. These biological vectors can carry target DNA or RNA molecules, inadvertently contaminating assay reagents or collection instruments.

In the context of LAMP assays, which are known for their extreme sensitivity and low specificity thresholds, such contamination can result in false-positive detections that misrepresent the actual presence of a pathogen or genetic trait in a given specimen. This risk is heightened in environments lacking stringent spatial controls or sterilization protocols. For instance, trace amounts of target nucleic acids transferred from a heavily infected neighboring plant can amplify robustly, thus creating misleading diagnostic outcomes. Furthermore, the iterative nature of field sampling - often performed sequentially with shared equipment - exacerbates this vulnerability unless rigorous decontamination steps are implemented between each sample. This environmental permeability underscores a key limitation of *in situ* diagnostics: while they offer speed and logistical convenience, they also operate within a matrix of uncontrollable biological noise.

In summary, although LAMP has been utilized in plant pathology, particularly its application in-field remains limited and technically constrained, falling short in terms of field-readiness, accessibility, and robustness. Furthermore, the method's inherent contamination risks, unless actively mitigated, threaten its utility in field-sampling.

### References

Gomez-Gutierrez & Goodwin, Loop-Mediated Isothermal Amplification for Detection of Plant Pathogens in Wheat (Triticum aestivum), Front. Plant Sci. (2022), 13 - 2022.
Harmon et al., A rapid PCR-based method for the detection of Magnaporthe oryzae from infected perennial ryegrass, Plant Disease (2003), 87: 1072-1076).
Khater et al., Biosensors for plant pathogen detection. Biosensors and Bioelectronics (2017), 93: 72-86.
Lopez et al., Are molecular tools solving the challenges posed by detection of plant pathogenic bacteria and viruses, Current Issues in Molecular Biology (2009), 11: 13-46.
Muthukumar et al., Loop-mediated isothermal amplification and its limit of detection for diagnostics of plant pathogens, Plant Protection Science (2024), 61(1).
Notomi et al. Loop-mediated isothermal amplification of DNA, Nucleic Acids Research (2000), Volume 28, Issue 12, Page e63.
Oliveira et al., Isothermal amplification of nucleic acids: the race for the next "gold standard". Frontiers in Sensors (2021), 2: 752600.
Pozharskiy et al., Molecular detection and sequencing of beet necrotic yellow vein virus and beet cryptic virus 2 in sugar beet from Kazakhstan, Front. Microbiol. (2024), 15 - 2024.

### Summary of the Invention

The above mentioned problem is solved in a first aspect by an *in situ* loop mediated isothermal amplification method for identifying at least one nucleic acid target sequence in at least one plant material, the method comprising the steps of: (a) providing and/or preparing a loop mediated isothermal amplification (LAMP) reaction mix comprising a set of primers, preferably comprising or consisting of six primers; (b) providing the at least one plant material and optionally providing an extraction kit to obtain the biological sample; comprising the followings steps: (i) excising the at least one plant material from at least one plant, preferably wherein the at least one plant material is a leaf, more preferably the leaf margin; and (ii) isolating the at least one plant material without cross-contamination from any other plant source; and (iii) mechanically disrupting the at least one plant material, preferably in combination with cell lysis; and (c) extracting at least one nucleic acid molecule from the at least one plant material, (d) performing a LAMP assay on the at least one nucleic acid molecule for allowing the detection of successful amplification of the respective target sequence amplified, (e) obtaining at least one test result, preferably wherein the test result is displayed in a lateral flow assay, (f) optionally: entering the at least one test result in a wireless mobile device, (g) optionally: allowing the wireless mobile device to transmit the at least one test result to a database, wherein georeferenced boundaries of field management units are stored in the database; wherein the at least one test result is available in less than one hour, preferably in less than 45 min, more preferably in less than 30 min and most preferably in less than 15 min.

One embodiment relates to the method of the first aspect, comprising the following additional steps: (i) providing a database that is provided on a server, wherein georeferenced boundaries of field management units, preferably are stored in the database, (ii) providing an assessment unit, comprising a processor, (iii) determining a current position of a wireless mobile device by using a global navigation satellite system (GNSS) receiver for establishing a position of the wireless mobile device, (iv) determining, by the assessment unit, a current field management unit, in which he wireless mobile device is located, based on the current position of the wireless mobile device and based on the georeferenced boundaries of the field management units stored in the database, (v) determining, by the assessment unit, one or more adjacent field management units, which are arranged adjacent to the current field management unit, based on the current position of the wireless mobile device and/or on the current field management unit and based on the georeferenced boundaries of the field management units stored in the database, (vi) storing the previous positions of the wireless mobile device and/or previous field management units, in which the wireless mobile device was located: wherein at least the following information of the current field management unit and of at least one adjacent field management unit, preferably of all field management units, is stored in the database: (a) status information of the field management unit; (b) required agricultural interventions.

One embodiment relates to the method according to the first aspect or the embodiment thereof, wherein the excising is performed by a punching part, preferably of a closure element, of a receptacle, preferably a reaction container.

One embodiment relates to the method according to the first aspect or any embodiment thereof, wherein the at least one nucleic acid molecule originates from at least one viral, bacterial, nematode or fungal plant pathogen, or plant gene, preferably a gene conferring resistance against the at least one plant pathogen.

One embodiment relates to the method according to the previous embodiment, wherein the at least one plant pathogen is selected from the group as defined in **Table 1,** preferably wherein the at least one plant pathogen is a *Beta spp.* pathogen, more preferably a *Beta vulgaris* pathogen, more preferably wherein the at least one pathogen is selected from at least one yellowing virus, including beet chlorosis virus (BChV), Beet mild yellowing virus (BMYV) and Beet yellows virus (BYV) and/or a fungal pathogen, including *Cercospora beticola.*

One embodiment relates to the method according to the first aspect or any embodiment thereof, wherein the method is at least partially, preferably completely, conducted *in situ* and/or in a non-laboratory environment, preferably in an agricultural field.

One embodiment relates to the method according to the first aspect or any embodiment thereof, wherein the method is a multiplexing isothermal detection method for detecting at least two, preferably at least three, more preferably for or more than four pathogens.

A second aspect relates to a system for obtaining at least one test result, wherein the at least one test result comprises detecting at least one target sequence in an *in situ* loop mediated isothermal amplification method according to any of the preceding claims, preferably a portable system, said system comprising: (i) optionally: a first receptacle, preferably a specimen bag; (ii) at least one reaction container comprising a primer set, a LAMP buffer solution, a strand-displacing DNA polymerase, optionally a reverse transcriptase, optionally uracil-DNA-glycosylase; and (iii) a detection device, preferably a lateral flow test strip, for obtaining the at least one test result; and (iv) at least one portable heating system, preferably heat block, wherein the at least one portable heating system achieves a temperature of up to 55°C, preferably up to 60°C, more preferably up to 65°C, most preferably at least 65°C; and (v) optionally: at least one mobile power source, preferably a high-performance power bank; and (vi) optionally: a wireless mobile device, comprising a control unit that is adapted to control said wireless mobile device to transmit information about the test result to a database.

One embodiment relates to the system according to the second aspect, wherein the primer set is pre-stored in the reaction container, wherein the primer set is provided attached to at least one coloured reaction ball, preferably wherein the coloured reaction ball additionally contains the LAMP buffer solution, the strand-displacing DNA polymerase, optionally the reverse transcriptase, and optionally the uracil-DNA-glycosylase, preferably in lyophilized form.

A third aspect relates to a primer set for detecting the presence or absence of a *Beta spp.* specific, preferably a *Beta vulgaris* specific, pathogen, for use in the method of the first aspect or any embodiment thereof and/or as part of the system according to the second aspect and the embodiment thereof, preferably for simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* wherein each of said at least one primer set comprises a forward primer, a reverse primer, a forward inner primer, a reverse inner primer, a loop forward primer and a loop reverse primer, wherein the primer sets are selected from: (i) a primer set for detecting BChV, comprising one primer each from the six groups selected from (i1) SEQ ID NO: 1 and 2, (i2) SEQ ID NO: 3 and 4, (i3) SEQ ID NO: 5 and 6, (i4)SEQ ID NO: 7 and 8, (i5) SEQ ID NO: 9 and 11, (i6) SEQ ID NO: 12 and 13, and/or (ii) a primer set for detecting BMYV, comprising one primer each from the six groups selected from (ii1) SEQ ID NO: 14, (ii2) SEQ ID NO: 15, (ii3) SEQ ID NO: 16 and 17, (ii4) SEQ ID NO: 18 and 19, (ii5) SEQ ID NO: 20, (ii6) SEQ ID NO: 21, and/or (iii) a primer set for detecting BYV, comprising one primer each from the six groups selected from (iii1) SEQ ID NO: 22 and 23, (iii2) SEQ ID NO: 24 and 25, (iii3) SEQ ID NO: 26 and 27, (iii4) SEQ ID NO: 28 and 29, (iii5) SEQ ID NO: 30 and 31, (iii6) SEQ ID NO: 32 and 33, and/or (iv) a primer set for detecting *Cercospora beticola,* comprising one primer each from the six groups selected from (iv1) SEQ ID NO: 34 and 35, (iv2) SEQ ID NO: 36 and 37, (iv3) SEQ ID NO: 38 and 39, (iv4) SEQ ID NO: 40 and 41, (iv5) SEQ ID NO: 42 and 43, and/or (iv6) SEQ ID NO: 44 and 45.

One embodiment relates to the primer set according to the third aspect comprising at least two, three, or all four primer sets of (i), (ii), (iii), or (iv) in combination with each other.

One embodiment relates to the primer set according to the third aspect or the embodiment thereof,, wherein the primer set for detecting BChV comprises the set of sequences as defined by SEQ ID NO: 2, 4, 6, 8, 11 and 13, and/or the primer set for detecting BMYV comprises the set of sequences as defined by SEQ ID NO: 14, 15, 17, 18, 20 and 21, and/or the primer set for detecting BYV comprises the set of sequences as defined by SEQ ID NO: 22, 24, 26, 28, 30 and 32, and/or wherein primer set for detecting *Cercospora beticola* comprises the set of sequences as defined by SEQ ID NO: 35, 37, 39, 41, 43 and 45.

One embodiment relates to the primer set according to the third aspect or any embodiment thereof, wherein at least one primer, preferably at least one forward inner or at least one reverse primer, comprises at least one label, preferably a 5'-label, preferably wherein the label is selected from the group of biotin, FITC and FAM.

A fourth aspect relates to a use of the system according to tthe second aspect or the embodiment thereof, for performing the loop mediated isothermal amplification method according to the first aspect or any embodiment thereof on the at least one target sequence.

One embodiment relates to the use according to the fourth aspect, wherein the result of the loop mediated isothermal amplification method is used to provide individual plants in a field identified as positive for at least one pathogen with a suitable individualized treatment.

### Brief Description of Drawings

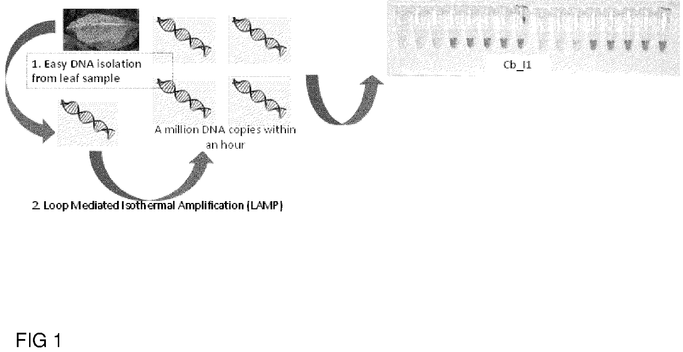

FIG 1: Detection limit results from loop mediated isothermal amplification (LAMP) primer sets.
   LAMP is used to amplify a specific DNA marker of *Cercospora beticola* at 65 °C. Incubation times for BY1 (BYV) and Cb_I1 = 30 min and BChV = 45 min. Concentrations of *C*. *beticola* = 1 to 10⁻⁶ ng/µl pure DNA of *C*. *beticola* strains cb150 and cb266. Concentrations of yellowing viruses = 1 to 10⁻⁶ ng/µl plant-virus mixed RNA of BChV (strains BC17 and BC18) and BYV (BY 76 and BY77). The last tube is negative control (PCR water). Plant extract negative controls as well as the primer set BM3new (BMYV) were tested separately.

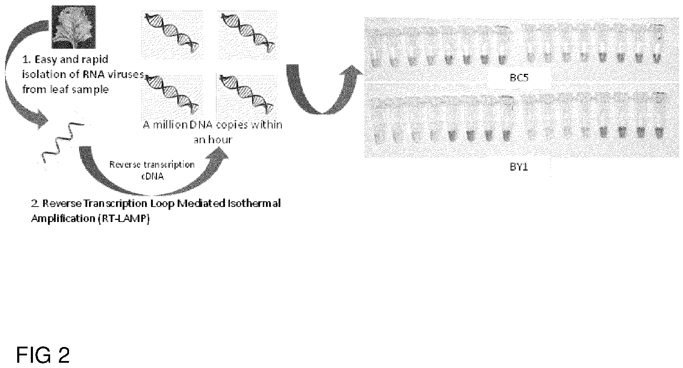

FIG 2: Detection limit results from reverse transcribed loop mediated isothermal amplification (RT-LAMP) primer sets.
   Incubation times for BY1 (BYV) and Cb_I1 = 30 min and BChV = 45 min. Concentrations of *C*. *beticola =* 1 to 10⁻⁶ ng/µl pure DNA of *C*. *beticola* strains cb150 and cb266. Concentrations of yellowing viruses = 1 to 10⁻⁶ ng/µl plant-virus mixed RNA of BChV (strains BC17 and BC18) and BYV (BY 76 and BY77). The last tube is negative control (PCR water). Plant extract negative controls as well as the primer set BM3new (BMYV) were tested separately.
FIG 3: Successful validation of LAMP tests based on LyoPrime WarmStart fluorescent LAMP/RT-LAMP Mix (with UDG).
   Yellowing viruses detection using LAMP based on LyoPrime WarmStart fluorescent LAMP/RT-LAMP Mix (with UDG) and Milenia HybriDetect (C = Control, BC = BChV, BM = BMYV and BY = BYV).
FIG 4: LAMP tests based on LyoPrime WarmStart fluorescent LAMP/RT-LAMP Mix (with UDG) for detection of *Cercospora.*
   The (green) triangle indicates the positive test result for a probe with early stage *Cercospora* infection, demonstrating high test sensitivity.
FIG 5: Representative LAMP assay tool kit and experimental workflow.

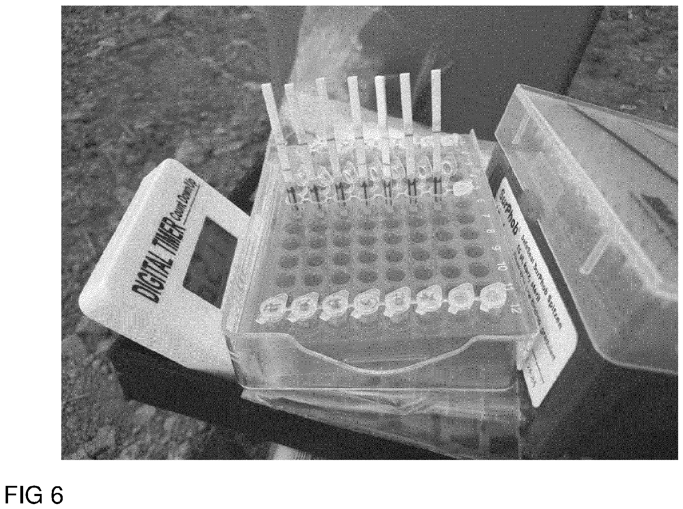

FIG 6: Pathogen detection by lateral flow assay.
   Successful in-field pathogen detection using lateral flow assay strips, shown here with attached filter papers from the procedure detailed in the Example 3.
FIG 7: Representative LAMP assay tool kit for cell lysis.
   The right panel depicts a set of reaction containers as part of an in situ LAMP assay tool kit. The outmost left reaction containers are shown as a close-up in the left panel, whereas the outmost left reaction container with the coloured cap contains a reaction ball (indicated by an arrow) for performing the LAMP assay.

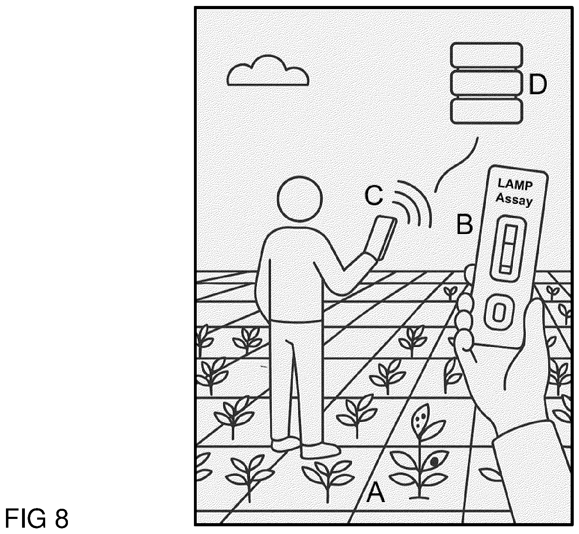

FIG 8: Schematic representation of an in-field LAMP assay with data transmission.

A pathogen-infected plant (A) is analyzed using a LAMP assay, with the test outcome visualized via a lateral flow assay (B). The result is subsequently transmitted by a wireless mobile device (D) to a central database (E).

### Brief Description of Sequences

| **Seq ID NO** | **Primer name** | **Seq ID NO** | **Primer name** |
|---|---|---|---|
| 1 | F3_BC1 x1 | 24 | B3_BY1 z2 |
| 2 | F3_BC5 x1 | 25 | B3_BY3 z2 |
| 3 | B3_BC1 x2 | 26 | FIP_BY1 z3 |
| 4 | B3_BC5 x2 | 27 | FIP_BY3 z3 |
| 5 | FIP_BC4 x3 | 28 | BIP_BY1 z4 |
| 6 | FIP_BC5 x3 | 29 | BIP_BY3 z4 |
| 7 | BIP_BC1 x4 | 30 | LoopF_BY1 z5 |
| 8 | BIP_BC5 x4 | 31 | LoopF_BY3 z5 |
| 9 | LoopF_BC1 x5 | 32 | LoopB_BY1 z6 |
| 10 | LoopF_BC1_2 | 33 | LoopB_BY3 z6 |
| 11 | LoopF_BC5 | 34 | F3_CB_C2 w1 |
| 12 | LoopB_BC1 x6 | 35 | F3_CB_I1 w1 |
| 13 | LoopB_BC5 x6 | 36 | B3_CB_C2 w2 |
| 14 | F3_BM1 y1 | 37 | B3_CB_I1 w2 |
| 15 | B3_BM1 | 38 | FIP_CB_C2 w3 |
| 16 | FIP_BM1 y3 | 39 | FIP_CB_I1 w3 |
| 17 | FIP_BM3 y3 | 40 | BIP_CB_C2 w4 |
| 18 | BIP_BM1 y4 | 41 | BIP_CB_I1 w4 |
| 19 | BIP_BM2 y4 | 42 | LoopF_CB_C2 w5 |
| 20 | LoopF_BM1 y5 | 43 | LoopF_CB_I1 w5 |
| 21 | LoopB_BM1 y6 | 44 | LoopB_CB_C2 w6 |
| 22 | F3_BY1 z1 | 45 | LoopB_CB_I1 w6 |
| 23 | F3_BY3 z1 | | |

### Brief Description of Sequences

### Definitions

A "lateral flow assay" is a paper- or membrane-based diagnostic method that uses capillary action to transport fluid samples across one or more detection zones on a lateral flow assay detection strip. As the sample migrates along the strip, it encounters labeled detection reagents and immobilized capture molecules; if the target analyte is present, a visible or measurable signal is generated. Control lines are typically integrated to verify proper flow and reagent functionality. Multiple formats and labeling techniques can be employed. For example, gold nanoparticles can be used as visible labels to detect biotinylated or fluorescently tagged analytes, and a test line may contain streptavidin to capture biotin-labeled targets. Additional lines or detection chemistries (e.g., anti-conjugate antibodies) can confirm the test's validity or enable quantitative readouts. These variations make LFAs adaptable to diverse applications ranging from clinical diagnostics to agricultural and environmental testing.

A "mobile power source" is a battery based device capable of delivering a consistent, high-current electrical supply to portable equipment, such as a field-deployable portable heating system, without reliance on fixed infrastructure. Built with robust power-management circuitry and high-capacity rechargeable cells, this type of power bank is engineered to maintain stable voltage and current levels over prolonged operational periods, making it particularly useful in remote or low-resource settings where reliable mains power is unavailable.

A "wireless mobile device" is defined as any portable, battery-powered communication and computing tool designed to connect to wireless networks, preferably cellular or Wi-Fi, without relying on fixed infrastructure. Preferably, it is engineered for convenient transport and on-demand connectivity, enabling voice, data, and multimedia exchanges in diverse settings, ranging from everyday personal use to specialized field applications.

A "portable heating system", e.g., a heat block, is a compact, self-contained device designed to maintain precise temperature levels for various applications. It is often powered by a mobile power source or standard electrical outlets, allowing it to be easily transported and used in remote or on-the-go scenarios that require controlled heating.

### Detailed Description

The present invention relates to isothermal nucleic acid amplification, particularly loop-mediated isothermal amplification (LAMP) assays, and their application for detecting pathogens in plants. Specifically, it addresses the challenges posed by existing LAMP assays in terms of storage, cross-contamination risks, and the requirement for lab infrastructure, thereby introducing an easy-to-use, field-compatible assay system. In addition, by combining real-time pathogen detection with a predictive software-assisted field mapping system, the invention significantly improves plant pathogen diagnostics, integrated plant protection strategies, disease monitoring, seed quality assessment, in-field agronomic consultancy, and plant breeding programs.

The present invention addresses the urgent need for robust and field-deployable diagnostic tools that empowers growers and agricultural professionals to implement timely disease management strategies to ultimately enhance crop protection and productivity.

According to a first aspect, the problem is solved by providing an *in situ* loop mediated isothermal amplification method for identifying at least one nucleic acid target sequence in at least one plant material, the method comprising the steps of: (a) providing and/or preparing a loop mediated isothermal amplification (LAMP) reaction mix comprising a set of primers, preferably comprising or consisting of six primers; (b) providing the at least one plant material and optionally providing an extraction kit to obtain the biological sample; comprising the followings steps: (i) excising the at least one plant material from at least one plant, preferably wherein the at least one plant material is a leaf, more preferably the leaf margin; and (ii) isolating the at least one plant material without cross-contamination from any other plant source; and (iii) mechanically disrupting the at least one plant material, preferably in combination with cell lysis; and (c) extracting at least one nucleic acid molecule from the at least one plant material, (d) performing a LAMP assay on the at least one nucleic acid molecule for allowing the detection of successful amplification of the respective target sequence amplified, (e) obtaining at least one test result, preferably wherein the test result is displayed in a lateral flow assay, (f) optionally: entering the at least one test result in a wireless mobile device, (g) optionally: allowing the wireless mobile device to transmit the at least one test result to a database, wherein georeferenced boundaries of field management units are stored in the database; wherein the at least one test result is available in less than one hour, preferably in less than 45 min, more preferably in less than 30 min and most preferably in less than 15 min.

Mechanical disruption of plant material may involve a variety of physical actions including crushing, grinding, shearing, or impact forces. These can be applied using a wide range of tools or devices known in the art.

During cell lysis, the disruption of the cellular membrane is augmented chemically, enzymatically, or thermally to ensure the complete release of intracellular components, especially nucleic acids and proteins that may be sequestered within organelles or bound to intracellular structures.

In certain embodiments, the LAMP assay may be performed as a Reverse Transcription Loop-Mediated Isothermal Amplification (RT-LAMP) assay, enabling the detection of RNA viruses or other RNA-based targets. In this configuration, the RT-LAMP assay incorporates a reverse transcription step within the same reaction tube, allowing for the simultaneous conversion of RNA into complementary DNA (cDNA) and its subsequent amplification under isothermal conditions. This is achieved using reverse transcriptase, which operates alongside strand-displacing DNA polymerase, eliminating the need for separate reverse transcription and amplification steps.

One embodiment relates to the method, comprising the following additional steps: (i) providing a database that is provided on a server, wherein georeferenced boundaries of field management units, preferably are stored in the database, (ii) providing an assessment unit, comprising a processor, (iii) determining a current position of a wireless mobile device by using a global navigation satellite system (GNSS) receiver for establishing a position of the wireless mobile device, (iv) determining, by the assessment unit, a current field management unit, in which he wireless mobile device is located, based on the current position of the wireless mobile device and based on the georeferenced boundaries of the field management units stored in the database, (v) determining, by the assessment unit, one or more adjacent field management units, which are arranged adjacent to the current field management unit, based on the current position of the wireless mobile device and/or on the current field management unit and based on the georeferenced boundaries of the field management units stored in the database, (vi) storing the previous positions of the wireless mobile device and/or previous field management units, in which the wireless mobile device was located: wherein at least the following information of the current field management unit and of at least one adjacent field management unit, preferably of all field management units, is stored in the database: (a) status information of the field management unit; (b) required agricultural interventions.

In the assessment, carried out by the assessment unit, it is preferably compared at least one stored characteristic of the wireless mobile device with information of a field management unit.

The GNSS receiver preferably is adapted to connect to a global navigation satellite system and receive position data therefrom. Preferably, the GNSS receiver is adapted to connect to two or more different systems, preferably to systems such as GPS and/or GLONASS and/or Galileo and/or BeiDou and/or further global navigation satellite systems. This is a particular advantage over existing solutions, which are bound to a single GNSS system.

The current field management unit is preferably understood to be the field management unit, in which the wireless mobile device is located at the time of the positioning of the wireless mobile device. The current field management unit can be determined based on the georeferenced boundaries of the field management units and on the current position of the wireless mobile device. From the information of the current position of the wireless mobile device and the georeferenced boundaries of the field management unit, it can be determined the current position of the wireless mobile device.

The current position of the wireless mobile device is preferably understood to be the position in which the wireless mobile device is located at the time of the positioning of the wireless mobile device.

An adjacent field management unit can preferably be understood as a field management unit that is arranged next to and/or neighbouring to the current field management unit. In particular an adjacent field management unit can be arranged adjoining to the current field management unit. A current field management unit can have one or more adjacent field management units. In particular, several adjacent field management units can be arranged adjoining to the current field management unit.

A previous position, as understood herein, can be understood as a position in which the wireless mobile device was located at a previous positioning of the mobile entity. The previous field management unit is preferably understood to be the field management unit, in which the wireless mobile device was located at the time of a positioning of the mobile entity. In particular, every current field management unit becomes a previous field management unit as a new current field management unit is determined. Thus, a previous field management unit preferably has been determined as a current field management unit is the past. Previous positions of the wireless mobile device in particular refer to positions of the wireless mobile device, in which the wireless mobile device was previously located. In particular, it can be understood that every current position of the wireless mobile device becomes a previous position of the wireless mobile device when the wireless mobile device moves further to a new current position. These previous positions can be stored in the database.

Agricultural interventions can preferably understood to be interferences to a field management unit, in particular interferences that have an effect on a field management unit, in particular on plants in a field management unit. Preferably, required agricultural interventions are interventions that are required for agricultural processes planned for a field management unit.

In the database, status information of a field management unit can be stored. Status information can for example comprise information on genetics of plants and/or seeds in the field management unit, and/or current status of plants and/or seeds in a field management unit such as for example blooming status or other information.

In preferred embodiments, the status information comprises detected infections by at least one plant pathogen and/or genetic resistance to at least one plant pathogen.

A schematic representation of the transmittance of an *in situ* LAMP test result to a database and leveraging of field-mapping information is depicted in FIG 8.

One embodiment relates to the method, wherein the excising is performed by a punching part, preferably of a closure element, of a receptacle, preferably a reaction container.

Preferably, the excising process - that is, the removal or separation of a portion of plant material - is achieved not by cutting, melting, or abrasion, but by punching. The punching mechanism may be part of a closure element, such as the snap cap of a reaction container, e.g., an Eppendorf tube, or any other suitable small-volume reaction container in laboratories known in the art.

In certain embodiments, the snap cap of a reaction container may be fitted with a punching protrusion, e.g., a pointed or bladed structure moulded or integrated into the inner side of the cap.

When the cap is closed with sufficient force, this protrusion may pierces and excises a portion of a thin sealing membrane (e.g., a foil or film covering the opening of the tube or inserted between the cap and the tube).

In certain embodiments the excised leaf segment may either fall into the tube, remain attached to the puncher, or be removed later depending on the system's design.

Advantageously, the integration of a punching part into the closure element of a receptacle, such as the cap of a reaction container, facilitates a simultaneous sealing and excising action that minimizes manual handling and external tool use. This design inherently supports operation within a closed system, which not only streamlines workflows but significantly reduces the risk of cross-contamination - particularly from external sources such as airborne particulates or neighbouring field plants. In agricultural or environmental sample processing, where genetic or biochemical material from adjacent specimens or field plants can compromise data integrity, this controlled excision method ensures a higher level of sample purity. By performing the excision internally during closure, exposure to surrounding contaminants is curtailed, making the technique especially valuable for sensitive diagnostic applications, such as LAMP-based detection of genetic material. Moreover, the ability to standardize the punching geometry enhances reproducibility, and the compact, integrated design promotes space efficiency, which is an important factor in a field-deployable system.

One embodiment relates to the method, wherein the at least one nucleic acid molecule originates from at least one viral, bacterial, nematode or fungal plant pathogen, or plant gene, preferably a gene conferring resistance against the at least one plant pathogen.

Plant resistance genes (also known as "R genes"), play a central role in the natural defense systems of numerous crops and wild plant species. They enable a plant to recognize invading pathogens, including bacteria, fungi, viruses, or nematodes, and initiate defense responses that limit or halt the spread of infection. A common structural feature of many resistance genes is the nucleotide-binding site and leucine-rich repeat (NBS-LRR) domain, which allows recognition of pathogen-derived molecules (effectors) and triggers a protective immune reaction, typically involving localized cell death at the infection site (Andersen et al., . Disease resistance mechanisms in plants. Genes (2018) 9:E339) Beyond NBS-LRR genes, there are other classes of R genes that enhance disease resistance through different mechanisms. For example, receptor-like proteins and receptor-like kinases (often denoted RLPs and RLKs) perceive effector molecules outside the plant cell, forming the first line of immune defense.

By harnessing natural or engineered resistance genes, plant breeders can develop varieties that resist prevalent diseases without relying heavily on chemical controls. Modern molecular techniques, including LAMP, enable quick identification of R genes and verification of their presence in breeding lines. As a result, plant scientists can screen large populations efficiently, support more sustainable farming practices, and contribute to global efforts aimed at enhancing crop productivity and resilience. Rz1 was the first major resistance gene deployed in virtually all commercial sugar beet varieties, providing effective control of beet necrotic yellow vein virus (BNYVV) under high disease pressure. However, this extensive reliance on a single gene created intense selection pressure on the virus population. Breeding programs responded by incorporating a second gene, Rz2, which confers resistance via a mechanism different from Rz1 and remains highly effective in areas where Rz1 has been circumvented by evolving virus populations. Experimental evidence has shown that homozygous Rz2-resistant sugar beet lines fail to support infection by BNYVV or the closely related beet soilborne mosaic virus (BSBMV), indicating a high degree of efficacy across these viral pathogens (Wetzel et al., The Beta vulgaris-derived resistance gene Rz2 confers broad-spectrum resistance against soilborne sugar beet-infecting viruses from different families by recognizing triple gene block protein 1, Molecular Plant Physiology (2021), 22, 7, 829-842.).

Certain embodiments relate to the method, wherein the at least one nucleic acid molecule originates from the Rz1 or Rz2 gene in sugar beet.

Advantageously, by using at least one primer set specific for Rz1 or Rz2 and performing a LAMP assay, it is possible to determine whether a given sugar beet sample carries this resistance gene. Detection of the nucleic acid molecule originating from a resistance gene may be combined with, or performed independently from, an assay that detects at least one nucleic acid molecule originating from at least one plant pathogen. This may allow rapid and accurate assessment of both disease presence and genetic resistance in a single, streamlined procedure.

One embodiment relates to the method, wherein the at least one plant pathogen is selected from the group as defined in **Table 1** , preferably wherein the at least one plant pathogen is a *Beta spp.* pathogen, more preferably a *Beta vulgaris* pathogen, more preferably wherein the at least one pathogen is selected from at least one yellowing virus, including beet chlorosis virus (BChV), Beet mild yellowing virus (BMYV) and Beet yellows virus (BYV) and/or a fungal pathogen, including *Cercospora beticola.*

**Table 1**

| **Pathogen** | **Disease** | **Pathogen type** |
|---|---|---|
| *Fusarium* spp. | *Fusarium root rot* / *Fusarium Yellows* | fungus |
| *Cercospora beticola* | *Cercospora Leaf spot* | fungus |
| *Aphanomyces cochlioides* | *Aphanomyces seedling* / *root rot* | fungus |
| *Rhizoctonia solani* | *Rhizoctonia seedling* / *root rot* | fungus |
| *Erysiphe betae* | *Powdery Mildew* | fungus |
| *Helicobasidium purpureum* | *Violet root rot* | fungus |
| *Uromyces betae* | *Beet rust* | fungus |
| *Sclerotium rolfsii* | *Sclerotium root rot* | fungus |
| *Stemphylium* spp. | *Stemphilium leaf spot* | fungus |
| *Peronospora farinosa f.sp. betae* | *Downy Mildew* | fungus |
| *Polymyxa betae* | *Vector BNYVV* | fungus |
| *Alternaria alternata* | *Alternaria leaf spot* | fungus |
| *Verticillium dahliae* | *Verticillium wilt* | fungus |
| Pseudomonas syringae | *Bacterial leaf spot* | bacterium |
| *Erwinia carotovora* | *Erwinia root rot* | bacterium |
| *Candidatus Arsenophonus phytopathogenicus* (SBR) | Syndrome des basses richesses | proteobacterium |
| *Candidatus Phytoplasma Solani* (RTD) | Rubbery Taproot disease | phytoplasma |
| BMYV | Beet Mild Yellowing Virus | virus |
| BChV | Beet Chlorosis Virus | virus |
| BYV | Beet Yelowing Virus | virus |
| BtMV | Beet Mosaic Virus | virus |
| BNYVV | Rhizomania | virus |
| BSBV | Beet soilborne virus | virus |
| BSBMV | Beet soilborne mosaic virus | virus |
| BCTV | Curly Top | virus |
| *Heterodera* spp. | Beet cyst nematodes | nematode |
| *Meloidogyne* spp. | Beet Gall nematodes | nematode |
| *Ditylenchus dipsaci* | Bulb nematode | nematode |

*Candidatus Phytoplasma solani* and the proteobacterium *Candidatus Arsenophonus phytopathogenicus* are pathogens known to be associated with two significant sugar beet diseases: "Syndrome Basses Richesses" (SBR, or "syndrome of low sugar content") and Rubbery Taproot Disease (RTD). In SBR, infected plants exhibit markedly reduced sugar content, while RTD is characterized by an abnormal, rubbery texture in the taproot. Both pathogens are transmitted by insect vectors, posing serious challenges for sugar beet cultivation and causing substantial economic losses.

One embodiment relates to the method according to the first aspect or any embodiment thereof, wherein the method is at least partially, preferably completely, conducted *in situ* and/or in a non-laboratory environment, preferably in an agricultural field.

One embodiment relates to the method, wherein the method is a multiplexing isothermal detection method for detecting at least two, preferably at least three, more preferably for or more than four pathogens.

Advantageously, these methods employ a specialized LAMP primer set designed with modifications that facilitate the distinct detection of multiple targets in a single reaction. Such modifications typically involve adding different fluorescent labels - each with a unique excitation and emission spectrum - and quenchers that suppress background signals. By assigning a specific fluorophore-quencher pair to each target, it becomes possible to track and distinguish multiple amplicons simultaneously. Examples of methodologies utilizing these modified primers include DARQ (Detection of Amplification by Release of Quenching), QUASR (Quenching of Unincorporated Amplification Signal Reporters), FLOS (Fluorescence of Loop primer upon Self-dequenching), guanine-based quenching approaches, and assimilation probes. These techniques collectively enhance the versatility and sensitivity of LAMP, enabling researchers to gain more comprehensive and rapid insights into the presence of diverse pathogens or genes of interest.

A plethora of methodologies employing multiplexing isothermal detection methods are known in the art, e.g., as described in Crego-Vincente et al., Multiplexing LAMP Assays: A Methodological Review and Diagnostic Application, Int J Mol Sci. (2024), 25(12): 6374.

A second aspect relates to a system for obtaining at least one test result, wherein the at least one test result comprises detecting at least one target sequence in an *in situ* loop mediated isothermal amplification method according to any of the preceding claims, preferably a portable system, said system comprising: (i) optionally: a first receptacle, preferably a specimen bag and/or a lysis container comprising at least one lysis buffer, (ii) at least one reaction container comprising a primer set, a LAMP buffer solution, a strand-displacing DNA polymerase, optionally a reverse transcriptase, optionally uracil-DNA-glycosylase; and (iii) a detection device, preferably a lateral flow test strip, for obtaining the at least one test result; and (iv) at least one portable heating system, preferably heat block, wherein the at least one portable heating system achieves a temperature of up to 55°C, preferably up to 60°C, more preferably up to 65°C, most preferably at least 65°C; and (v) optionally: at least one mobile power source, preferably a high-performance power bank; and (vi) optionally: a wireless mobile device, comprising a control unit that is adapted to control said wireless mobile device to transmit information about the test result to a database.

In some embodiments, the lysis buffer is optimized for different pathogen types. For RNA viruses, the lysis buffer may comprise guanidine hydrochloride, Triton X-100, and a Tris-based solution to facilitate effective viral RNA extraction, but not limited thereof. For fungal DNA, the lysis buffer may comprise NaCl, polyvinylpyrrolidone (PVP), Tween, and a Tris-based solution, ensuring efficient fungal DNA extraction, but not limited thereof.

In some embodiments, the primers within the detection kit may be provided in a lyophilized form to enhance stability and shelf life, making them suitable for extended storage and field deployment. Alternatively, the primers may be supplied in a solution or mixed solution form for immediate use, facilitating convenient field applications.

In some embodiments, the system may further include a micropipette for precise sample handling and reagent transfer, improving accuracy and reproducibility in field diagnostic testing.

In some embodiments, the detection kit is configured to accommodate mechanical pretreatment of samples. Sample processing may be performed using a plant grinding machine for efficient tissue disruption. Alternatively, plant material may be placed into a pre-filled lysis buffer bag, where it can be macerated using a pen cap or the end of a pen body, providing a simple, equipment-free method for sample preparation in field conditions.

In some embodiments, the detection kit may be applicable to both direct and indirect sample analysis. Direct samples may include sugar beet leaves, roots, and seeds, which are commonly tested for pathogen presence. Indirect samples, such as soil, insect vectors (e.g., aphids), weeds, sugar beet flowers, and other plant species, may also be analyzed to assess potential reservoirs and transmission sources of pathogens. This expanded sampling capability may be particularly advantageous for epidemiological studies and monitoring pathogen spread.

In preferred embodiments, the detection kit may be designed for field-ready use, eliminating the requirement for laboratory infrastructure. This feature may enhance accessibility for agronomists, plant pathologists, and farmers, enabling real-time pathogen detection and monitoring.

In some embodiments, the detection kit may offer a rapid and cost-effective testing solution, allowing for timely decision-making in disease control strategies and reducing crop losses. The streamlined workflow of the kit may minimize reliance on complex laboratory procedures and specialized personnel, making it suitable for broad agricultural applications.

In some embodiments, the detection kit may be utilized beyond routine plant pathogen diagnostics and integrated into surveillance programs. The ability to analyze indirect samples may facilitate vector monitoring and the identification of alternative pathogen sources, contributing to improved disease management strategies.

In one embodiment, there is provided a detection kit, wherein each primer or primer set is contained in one container or compartment in a lyophilized form, or wherein each primer or primer set is part of a solution or mixed solution, preferably in lyophilized form.

One embodiment relates to the system according to the second aspect, wherein the primer set is pre-stored in the reaction container, wherein the primer set is provided attached to at least one coloured reaction ball, preferably wherein the coloured reaction ball additionally contains the LAMP buffer solution, the strand-displacing DNA polymerase, optionally the reverse transcriptase, and optionally the uracil-DNA-glycosylase, preferably in lyophilized form.

By pre-storing the primer set on at least one coloured reaction ball, the LAMP assay setup becomes significantly more convenient and user-friendly. The coloured reaction ball serves as a visual indicator, simplifying assay preparation by removing the need to measure or transfer small reagent volumes individually. Incorporating additional components such as the LAMP buffer solution, the strand-displacing DNA polymerase, optional reverse transcriptase, and optionally uracil-DNA-glycosylase, preferably a complete master mix, within the reaction ball, preferably in a lyophilized form, further reduces the potential for pipetting errors and contamination. The assay can then be initiated by simply rehydrating the reaction ball under appropriate conditions, which is especially beneficial in *in situ* field-testing scenarios. This streamlined approach minimizes the time and technical expertise needed for assay setup while preserving the reliability and performance of the reaction components.

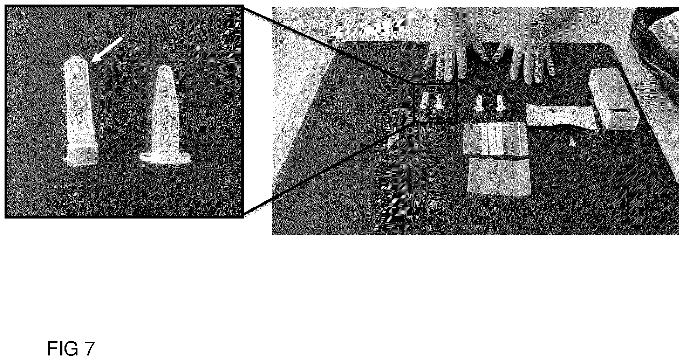

A photographic depiction of a reaction ball as part of a LAMP assay toolkit is displayed in FIG 7.

A third aspect relates to a primer set for detecting the presence or absence of a *Beta spp.* specific, preferably a *Beta vulgaris* specific, pathogen, for use in the method of the first aspect or any embodiment thereof and/or as part of the system according to the second aspect and the embodiment thereof, preferably for simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* wherein each of said at least one primer set comprises a forward primer, a reverse primer, a forward inner primer, a reverse inner primer, a loop forward primer and a loop reverse primer, wherein the primer sets are selected from: (i) a primer set for detecting BChV, comprising one primer each from the six groups selected from (i1) SEQ ID NO: 1 and 2, (i2) SEQ ID NO: 3 and 4, (i3) SEQ ID NO: 5 and 6, (i4)SEQ ID NO: 7 and 8, (i5) SEQ ID NO: 9 and 11, (i6) SEQ ID NO: 12 and 13, and/or (ii) a primer set for detecting BMYV, comprising one primer each from the six groups selected from (ii1) SEQ ID NO: 14, (ii2) SEQ ID NO: 15, (ii3) SEQ ID NO: 16 and 17, (ii4) SEQ ID NO: 18 and 19, (ii5) SEQ ID NO: 20, (ii6) SEQ ID NO: 21, and/or (iii) a primer set for detecting BYV, comprising one primer each from the six groups selected from (iii1) SEQ ID NO: 22 and 23, (iii2) SEQ ID NO: 24 and 25, (iii3) SEQ ID NO: 26 and 27, (iii4) SEQ ID NO: 28 and 29, (iii5) SEQ ID NO: 30 and 31, (iii6) SEQ ID NO: 32 and 33, and/or (iv) a primer set for detecting *Cercospora beticola,* comprising one primer each from the six groups selected from (iv1) SEQ ID NO: 34 and 35, (iv2) SEQ ID NO: 36 and 37, (iv3) SEQ ID NO: 38 and 39, (iv4) SEQ ID NO: 40 and 41, (iv5) SEQ ID NO: 42 and 43, and/or (iv6) SEQ ID NO: 44 and 45.

One embodiment relates to the primer set comprising at least two, three, or all four primer sets of (i), (ii), (iii), or (iv) in combination with each other.

In yet another embodiment, there is provided a primer set, wherein at least one primer, preferably at least one forward inner or at least one reverse primer, comprises at least one label, preferably a 5'-label, preferably wherein the label is selected from the group of biotin, FITC and FAM.

In some embodiments, dual-labeled primers may be used to create FRET (Förster Resonance Energy Transfer)-based probes, allowing highly specific signal generation and reducing false-positive results in fluorescence-based detection systems.

In some embodiments, the labeled primer set may be specifically optimized for lateral flow assays (LFAs), where biotin- or FITC-labeled amplicons can be detected using test strips, eliminating the need for specialized detection equipment in field applications.

In some embodiments, multiplex detection strategies may utilize differentially labeled primers, each targeting distinct pathogens, allowing for the simultaneous detection of multiple sugar beet pathogens in a single reaction through differential fluorescence readout or colorimetric detection.

In some embodiments, the primer set may be adapted for automated detection systems, where labeled amplicons are detected using microfluidic devices or portable fluorescence readers, enhancing scalability and high-throughput diagnostic applications.

In some embodiments, the labeled primers may be compatible with isothermal amplification techniques beyond LAMP, including recombinase polymerase amplification (RPA) and nucleic acid sequence-based amplification (NASBA), broadening their applicability for nucleic acid-based pathogen detection.

In some embodiments, the primer set may include labels that provide colorimetric, chemiluminescent, or electrochemical signal output, making them adaptable to biosensor-based detection platforms.

One embodiment relates to the primer set, wherein the primer set for detecting BChV comprises the set of sequences as defined by SEQ ID NO: 2, 4, 6, 8, 11 and 13, and/or the primer set for detecting BMYV comprises the set of sequences as defined by SEQ ID NO: 14, 15, 17, 18, 20 and 21, and/or the primer set for detecting BYV comprises the set of sequences as defined by SEQ ID NO: 22, 24, 26, 28, 30 and 32, and/or wherein primer set for detecting *Cercospora beticola* comprises the set of sequences as defined by SEQ ID NO: 35, 37, 39, 41, 43 and 45.

The primer sets are selected from specific sequences associated with each pathogen, ensuring high specificity and sensitivity. For example, the primer set for detecting BChV is defined by specific SEQ ID numbers for each of the six primers.

One embodiment relates to the primer set, wherein at least one primer, preferably at least one forward inner or at least one reverse primer, comprises at least one label, preferably a 5'-label, preferably wherein the label is selected from the group of biotin, FITC and FAM.

A fourth aspect relates to a use of the system, for performing the loop mediated isothermal amplification method on the at least one target sequence.

One embodiment relates to the use of the system, wherein the result of the loop mediated isothermal amplification method is used to provide individual plants in a field identified as positive for at least one pathogen with a suitable individualized treatment.

Once a plant tests positive for pathogen presence via LAMP, the suitable individualized treatment depends on the pathogenesis caused by said pathogen. Localized chemical application involves applying fungicides, bactericides, or insecticides only to those plants that tested positive, optionally extending to their immediate neighbours if they present a high risk of infection. This approach minimizes chemical usage across the field and helps preserve beneficial organisms.

Soil and nutrient adjustments become critical when the pathogen thrives under specific environmental conditions. Correcting issues such as imbalanced pH or inadequate nutrient levels around infected plants can significantly reduce disease progression. When excessive moisture favours pathogen spread, irrigation schedules and methods can be adjusted to keep soil or foliage dryer in the vicinity of infected plants.

In cases involving foliar pathogens, physical methods such as pruning and removing infected plant tissue help limit further transmission within and between plants. Such material should be disposed of properly to avoid reinfection. After these measures are in place, re-testing the plants with LAMP or other diagnostics validates the effectiveness of the interventions, allowing timely adjustments if the pathogen shows signs of resurgence.

### Examples

### Example 1: Detection of Yellowing Viruses Using LAMP Primers

### Material & Methods

### LAMP primer design for BChV, BMYV, BYV and Cercospora beticola

RNA of BChV, BMYV and BYV were extracted from reference infected sugar beet plants growing the KWS insect rearing chamber and the leaf samples from yellowing virus monitoring project 2022 extracted using Quick-RNA^{™} Miniprep Kit (Zymo Research, Freiburg, Germany) according to the manufacturing protocol. DNA of *Cercospora beticola* were extracted from mycelium of the reference isolates of our own KWS culture collection at Department of Phytopathology using (DNeasy Plant Pro Kit, Qiagen, Hilden, Germany). The RNA was transcribed to cDNA using LunaScript^{®} RT Master Mix (New England Biolabs, Ipswich, MA, USA). Genome of BChV, BMYV, BYV and *Cercospora beticola* was partly amplified with PCR and sequenced at Microsynth Seqlab (Göttingen, Germany). Additional sequencing data from BChV, BMYV, BYV and *Cercospora beticola* were also retrieved from NCBI GenBank. All sequencing data from each target were aligned together using ugene software version 46.0. The obtained consensus sequences were used for LAMP primer design (each primer set comprises 6 primers: B3, F3, BIP, FIP, LoopF and LoopB) in LAMP designer software (PREMIER Biosoft, San Francisco, CA, USA) and NEB LAMP design online tool (New England Biolabs, Ipswich, MA, USA). Generated primer sets were prescreening using reference BChV, BYV and BMYV RNA standards produced from infected sugar beet plants growing the KWS insect rearing chambers or *Cercospora beticola* reference DNA standard. The LAMP assays were conducted using WarmStart^{®} Colorimetric LAMP 2X Master Mix (DNA & RNA) (New England Biolabs, Ipswich, MA, USA). The potential primer sets which were specific to BChV (Beet chlorosis virus) were used for further tests for specificity and sensitivity.

### Specificity and sensitivity tests

Three to five high potential primer sets per each targeted pathogens were tested for specificity and sensitivity using standard RNA or DNA dilutions (10⁰ to 10⁻⁶ ng/µl total RNA or DNA) from common viral (BChV, BYV, BMYV and BtMV), bacterial (Candidatus Phytopasma solani and Candidatus Arsenophonus phytopathogenicus) and fungal (*Cercospora* spp. and *Fusarium* spp.) pathogens of sugar beet. The LAMP assays were conducted using WarmStart^{®} Colorimetric LAMP 2X Master Mix (DNA & RNA) (New England Biolabs, Ipswich, MA, USA) according to the manufacturing recommendations. The incubation times were tested at 15, 20, 25, 30, 45, 60 and 90 mins. The sensitivity of detection were compared with the results from the magnetic induction cycler qPCR (Bio Molecular Systems, Upper Coomera, and Australia). The primer sets which showed the best results was used for accuracy and repeatability tests.

### Results and Discussion

Four LAMP primer sets were successfully designed and showed sufficient, high or very high potential for detection of yellowing viruses (BMYV (primer set: **BM3,** sufficient)/BYV (primer set: **BY1,** very high)/BChV (primer set: **BC5,** high)) and *C. beticola* (primer set: **Cb_I1,** high) (FIG 1).

Sequence data from in-house facilities strongly contributed to the achievement of generating the new LAMP primers. The efficient detection threshold (sensitivity) was set at 10 to 100 times lower compared with the standard real-time PCR (i.e. total RNA concentration for real-time PCR 10⁻⁵ ng/µl and for LAMP 10⁻⁴ to 10⁻³ ng/µl).

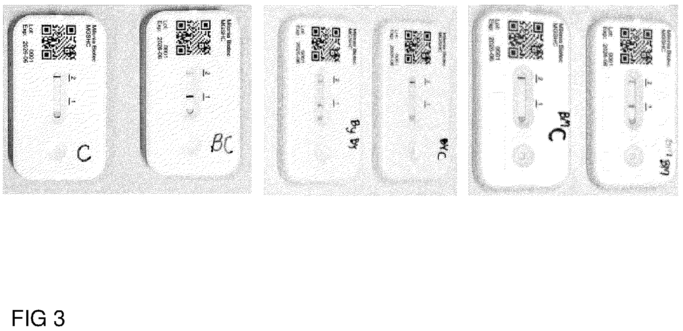

The specificity test included many common sugar beet pathogens encompassing viruses, fungi and bacteria showed that the developed LAMP primers are specific. The primer sets BY1 and Cb_I1 showed stable results with an incubation period of 30 to 45 min (FIG 3).

The use of LAMP primers for *C. beticola* (Cb_I1) lead to 30 to 45 min (50 to 66.7%) faster results compared to 90 min using the *published* LAMP primers (Shrestha et al., 2020). This was achieved by using 6 LAMP primers for *C*. *beticola,* by incorporating 2 efficient Loop primers, instead of only 4 LAMP primers according to the published protocol (Shrestha et al., 2020). The BC5 primer set shows stable results after 45 min (FIG 3).

### Example 2: Detection of Cercospora beticola using LAMP Primers

### Materials & Methods

### Accuracy and Repeatability Test

The accuracy of the BChV, BMYV, and BYV LAMP primers for detecting yellowing viruses in 75 field-collected samples was evaluated by comparing the results with standard protocols using real-time PCR (Mahillon et al., Virus Yellows and Syndrome "Basses Richesses" in Western Switzerland: A Dramatic 2020 Season Calls for Urgent Control Measures, pathogens (2022), 11, 885) for BChV and BYV. Two sensitivity levels of real-time PCR were used in this experiment: 10⁻⁴ ng/µl (100 times less sensitive) and 10⁻³ ng/µl (10 times less sensitive) total RNA. Accuracy tests were also conducted in comparison with standard conventional PCR (Mahillon et al., 2022) and ELISA. The experiment was repeated twice, and the consistency of the results was measured as repeatability (%).

### Results and Discussion

The results for the accuracy rates of the LAMP assays were promising. The accuracy for LAMP BYV was 100% at 10⁻³ ng/µl total RNA and 91.3% at 10⁻⁴ ng/µl total RNA. For LAMP BChV, the accuracy was 98.7% at 10⁻³ ng/µl total RNA and 86% at 10⁻⁴ ng/µl total RNA. LAMP BMYV showed an accuracy of 88% at 10⁻³ ng/µl total RNA **(Table 2** and accuracy and repeatability Excel file). The accuracy of LAMP assays with the newly developed LAMP primers was significantly higher than that of conventional PCR (24-82%) and ELISA (25% for BYV). **Table 2** shows comparisons of accuracy rates (as compared with real-time PCR at 10⁻³ ng/µl plant-virus mixed RNA, 100 time less sensitive) obtained by LAMP (with new primer sets), conventional PCR and ELISA (original data are provided in excel file). NA = not accessible.

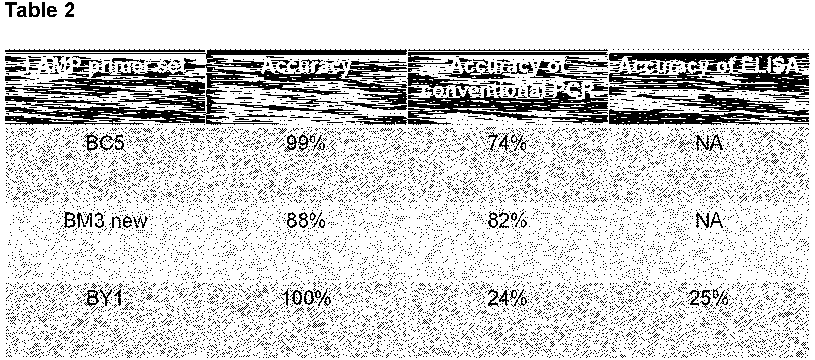

It should be noted that ELISA cannot differentiate between BChV and BMYV, so this test was not performed for these viruses. The repeatability rates of the LAMP assays ranged from 83% to 89%.

Repeatability could be improved by performing two technical replicates for each test. However, most of the uncertain samples had low to very low viral concentrations.

### Example 3: Validation of LyoPrime WarmStart fluorescent LAMP/RT-LAMP Mix (with UDG) (New England Biolabs, UK)

The aims of this experiment were to test and validate the LyoPrime WarmStart fluorescent LAMP/RT-LAMP Mix (with UDG) for detection of three important yellowing viruses in sugar beet (Beet yellows virus (BYV), Beet mild yellowing virus (BMYV) and Beet chlorosis virus (BChV)) and Cercospora using the established LAMP primers (BC5 for BChV, BM3 for BMYV, BY1 for BYV and CBI1 for Cercospora). The advantages of LyoPrime WarmStart fluorescent LAMP/RT-LAMP Mix (with UDG) are (i) it can be kept at room temperature, (ii) it can be mixed with primers during the lyophilized process and (iii) it can be detected also with real-time PCR machine. Moreover, when the LAMP assays are used together with the lateral flow assays, the color indicator is unnecessary.

### Material and methods

Nineteen RNA samples with known viral concentrations (determined by RT-qPCR) according to the KWS standard protocol were used. One PCR water sample was included as a negative control. LAMP assays were prepared in a similar manner to the protocols from the WarmStart^{®} Colorimetric LAMP 2X Master Mix (DNA & RNA), but using LyoPrime WarmStart fluorescent LAMP/RT-LAMP Mix (with UDG) instead (see the general LAMP assay procedure below). The LAMP reaction volume was 25 µL, and the incubation time ranged from 20 to 25 minutes at 65°C. A portable heat block was used. After incubation, the LAMP product was analyzed using the Milenia HybriDetect, and results were recorded after 5 minutes.

Two sets of leaf samples (leaf discs with a 5 mm diameter containing 0, 1, or 2 lesions of *Cercospora*-infected leaves) were used. An additional sample that tested negative for *Cercospora* was included as a negative control. LAMP assays were prepared in a similar manner to the protocols from the WarmStart^{®} Colorimetric LAMP 2X Master Mix (DNA & RNA), but using LyoPrime WarmStart fluorescent LAMP/RT-LAMP Mix (with UDG) instead. The LAMP reaction volume was 25 µL, and the incubation time was up to 30 minutes at 65°C. A portable heat block was used. After incubation, the LAMP product was analyzed using the Milenia HybriDetect, and results were recorded after 5 minutes. The portable heat block was used. After the incubation, the LAMP product was analyzed with the Milenia HybriDetect and the results were recorded after 5 mins.

### In situ LAMP assay procedure

A single LAMP reaction ball was added to Tube 1 (see FIG 7), which contained sterile PCR-grade water. The term "LAMP reaction ball" refers to a reaction ball containing all essential LAMP reagents (e.g., primers, polymerase, and buffer), required for the amplification reaction in lyophilized form, as a master mix. Preferably the reaction ball is coloured depending on the specific set of primers.

Two alternative were used for nucleic acid extraction:
1) A plant sample (one leaf disc, cut with a 1.5 mL tube as a guide) was placed into Tube 3 containing a metal ball and lysis buffer. The tube was shaken thoroughly to disrupt the tissue. Using filter paper, nucleic acids were collected from the tube six times (each pass of the paper saturated it with the lysate). The filter paper was then washed in Wash Buffer (Tube 4) by dipping it 30 times.
2) Alternatively, a plant sample (one leaf disc, cut with a 1.5 mL tube as a guide) was placed in a grinding bag. The lysis buffer from Tube 3 was poured into the bag. The sample was manually ground with gentle external force (e.g., using a pen). Filter paper was used to collect the nucleic acids six times, and it was then washed in Wash Buffer (Tube 4) by dipping it 30 times.

After the nucleic acid extraction step, the washed filter paper was transferred into Tube 1 (containing the LAMP reaction ball and sterile PCR-grade water) by dipping it into the solution 15 times. The tube was incubated at 65°C for 20 minutes, up to a maximum of 30 minutes. After this step, the filter paper was discarded.

A lateral flow buffer and lateral flow strip were prepared by adding 150 µL of the buffer (Solution 5) to a 200 µL test strip. The entire contents of Tube 1 (the LAMP reaction mix) were then transferred to the lateral flow strip. The lateral flow assay was allowed to develop for five minutes. The results were read and recorded based on the appearance of the test and control lines on the strip.

### Results and discussion

The results demonstrate the successful validation of LAMP tests using the LyoPrime WarmStart fluorescent LAMP/RT-LAMP Mix (with UDG) (FIG 3). A high level of consistency was observed between LAMP tests based on LyoPrime WarmStart fluorescent LAMP/RT-LAMP Mix (with UDG) and qPCR results. **Table 3** shows a comparison of LAMP tests based on LyoPrime WarmStart fluorescent LAMP/RT-LAMP Mix (with UDG) (Lyo) and qPCR results for detection of three yellowing viruses. Green and orange color highlights indicate consistent and inconsistent results of qPCR and LAMP assays, respectively.

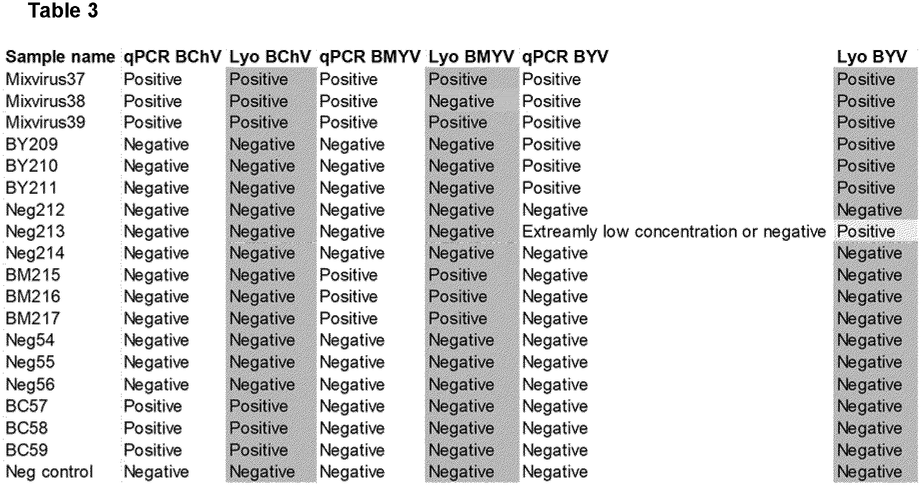

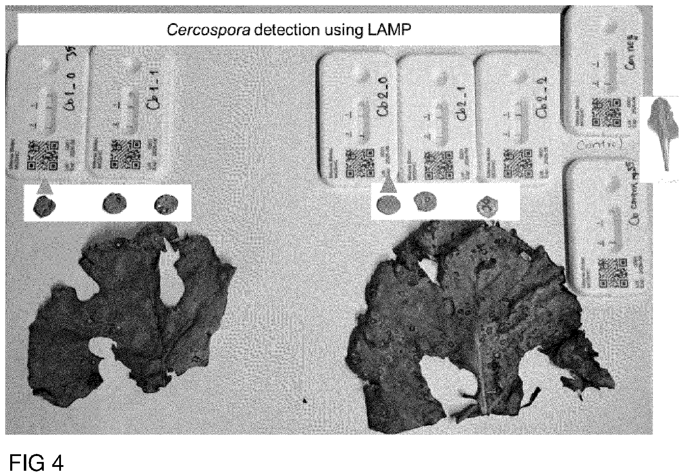

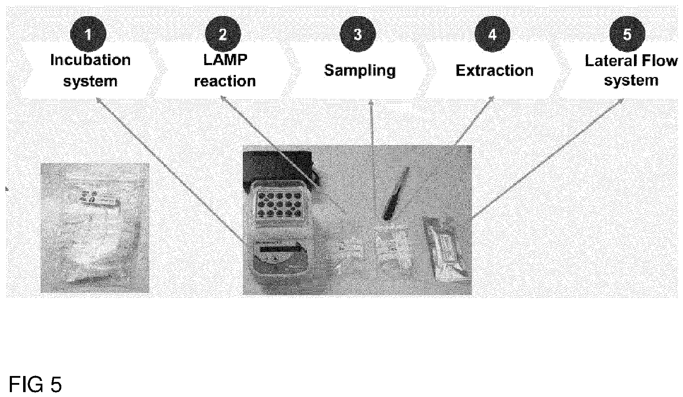

Overall, the findings confirm that the LyoPrime WarmStart fluorescent LAMP/RT-LAMP Mix (with UDG) is compatible with the developed primers and the LAMP assay protocol for detecting yellowing viruses. It can serve as a suitable alternative to the WarmStart^{®} Colorimetric LAMP 2X Master Mix (DNA & RNA). LAMP tests using the LyoPrime WarmStart fluorescent LAMP/RT-LAMP Mix (with UDG) were successfully applied to detect *Cercospora* in all infected sugar beet leaves, including leaf discs containing 0, 1, and 2 lesions of *Cercospora* symptoms (FIG 4). The negative control remained negative after a 30-minute incubation. These results indicate that LAMP tests based on the LyoPrime WarmStart fluorescent LAMP/RT-LAMP Mix (with UDG) are highly sensitive and can serve as a suitable alternative to the WarmStart^{®} Colorimetric LAMP 2X Master Mix (DNA & RNA). Early detection is achievable with a 30-minute incubation at 65°C. A prototypical LAMP assay toolkit is shown in FIG 5.

## Claims

1. An *in situ* loop mediated isothermal amplification method for identifying at least one nucleic acid target sequence in at least one plant material, the method comprising the steps of:
(a) providing and/or preparing a loop mediated isothermal amplification (LAMP) reaction mix comprising a set of primers, preferably comprising or consisting of six primers;
(b) providing the at least one plant material and optionally providing an extraction kit to obtain the biological sample; comprising the followings steps:
(i) excising the at least one plant material from at least one plant, preferably wherein the at least one plant material is a leaf, more preferably the leaf margin; and
(ii) isolating the at least one plant material without cross-contamination from any other plant source; and
(iii) mechanically disrupting the at least one plant material, preferably in combination with cell lysis; and
(c) extracting at least one nucleic acid molecule from the at least one plant material;
(d) performing a LAMP assay on the at least one nucleic acid molecule for allowing the detection of successful amplification of the respective target sequence amplified;
(e) obtaining at least one test result, preferably wherein the test result is displayed in a lateral flow assay;
(f) optionally: entering the at least one test result in a wireless mobile device;
(g) optionally: allowing the wireless mobile device to transmit the at least one test result to a database, wherein georeferenced boundaries of field management units are stored in the database;
wherein the at least one test result is available in less than one hour, preferably in less than 45 min, more preferably in less than 30 min and most preferably in less than 15 min.

2. The method of claim 1, comprising the following additional steps:
(i) providing a database that is provided on a server, wherein georeferenced boundaries of field management units, preferably are stored in the database,
(ii) providing an assessment unit, comprising a processor,
(iii) determining a current position of a wireless mobile device by using a global navigation satellite system (GNSS) receiver for establishing a position of the wireless mobile device,
(iv) determining, by the assessment unit, a current field management unit, in which he wireless mobile device is located, based on the current position of the wireless mobile device and based on the georeferenced boundaries of the field management units stored in the database,
(v) determining , by the assessment unit, one or more adjacent field management units, which are arranged adjacent to the current field management unit, based on the current position of the wireless mobile device and/or on the current field management unit and based on the georeferenced boundaries of the field management units stored in the database,
(vi) storing the previous positions of the wireless mobile device and/or previous field management units, in which the wireless mobile device was located;
wherein at least the following information of the current field management unit and of at least one adjacent field management unit, preferably of all field management units, is stored in the database:
(a) status information of the field management unit,
(b) required agricultural interventions.

3. The method according to claims 1 or 2, wherein the excising is performed by a punching part, preferably of a closure element, of a receptacle, preferably a reaction container.

4. The method according to any of the preceding claims, wherein the at least one nucleic acid molecule originates from at least one viral, bacterial, nematode or fungal plant pathogen, or plant gene, preferably a gene conferring resistance against the at least one plant pathogen.

5. The method according to claim 4, wherein the at least one plant pathogen is selected from the group as defined in **Table 1,** preferably wherein the at least one plant pathogen is *a Beta spp.* pathogen, preferably a *Beta vulgaris* pathogen, preferably wherein the at least one pathogen is selected from at least one yellowing virus, including beet chlorosis virus (BChV), Beet mild yellowing virus (BMYV) and Beet yellows virus (BYV) and/or a fungal pathogen, including *Cercospora beticola.*

6. The method according to any of the preceding claims, wherein the method is at least partially, preferably completely, conducted *in situ* and/or in a non-laboratory environment, preferably in an agricultural field.

7. The method according to any of the preceding claims, wherein the method is a multiplexing isothermal detection method for detecting at least two, preferably at least three, more preferably for or more than four pathogens.

8. A system for obtaining at least one test result, wherein the at least one test result comprises detecting at least one target sequence in an *in situ* loop mediated isothermal amplification method according to any of the preceding claims, preferably a portable system, said system comprising:
(i) optionally: a first receptacle, preferably a specimen bag;
(ii) at least one reaction container comprising a primer set, a LAMP buffer solution, a strand-displacing DNA polymerase, optionally a reverse transcriptase, optionally uracil-DNA-glycosylase; and
(iiii) a detection device, preferably a lateral flow test strip, for obtaining the at least one test result; and
(iv) at least one portable heating system, preferably heat block, wherein the at least one portable heating system achieves a temperature of up to 55°C, preferably up to 60°C, more preferably up to 65°C, most preferably at least 65°C; and
(v) optionally: at least one mobile power source, preferably a high-performance power bank; and
(vi) optionally: a wireless mobile device, comprising a control unit that is adapted to control said wireless mobile device to transmit information about the test result to a database.

9. The system according to claim 8, wherein the primer set is pre-stored in the reaction container, wherein the primer set is provided attached to at least one coloured reaction ball, preferably wherein the coloured reaction ball additionally contains the LAMP buffer solution, the strand-displacing DNA polymerase, optionally the reverse transcriptase, and optionally the uracil-DNA-glycosylase, preferably in lyophilized form.

10. A primer set for detecting the presence or absence of a *Beta spp.* specific, preferably *a Beta vulgaris* specific, pathogen, for use in the method of claims 1 to 7 and/or as part of the system according to claim 8 or 9, preferably for simultaneously detecting at least two *Beta spp.* specific, preferably a *Beta vulgaris* specific, pathogens, wherein the pathogen is selected from at least one yellowing virus, including beet chlorosis virus (BChV), Beet mild yellowing virus (BMYV) and Beet yellows virus (BYV) and/or a fungal pathogen, including *Cercospora beticola,* wherein each of said at least one primer set comprises a forward primer, a reverse primer, a forward inner primer, a reverse inner primer, a loop forward primer and a loop reverse primer, wherein the primer sets are selected from:
(i) a primer set for detecting BChV, comprising one primer each from the six groups selected from
(i1) SEQ ID NO: 1 and 2,
(i2) SEQ ID NO: 3 and 4,
(i3) SEQ ID NO: 5 and 6,
(i4) SEQ ID NO: 7 and 8,
(i5) SEQ ID NO: 9 and 11,
(i6) SEQ ID NO: 12 and 13, and/or
(ii) a primer set for detecting BMYV, comprising one primer each from the six groups selected from
(ii1) SEQ ID NO: 14,
(ii2) SEQ ID NO: 15,
(ii3) SEQ ID NO: 16 and 17,
(ii4) SEQ ID NO: 18 and 19,
(ii5) SEQ ID NO: 20,
(ii6) SEQ ID NO: 21, and/or
(iii) a primer set for detecting BYV, comprising one primer each from the six groups selected from
(iii1) SEQ ID NO: 22 and 23,
(iii2) SEQ ID NO: 24 and 25,
(iii3) SEQ ID NO: 26 and 27,
(iii4) SEQ ID NO: 28 and 29,
(iii5) SEQ ID NO: 30 and 31,
(iii6) SEQ ID NO: 32 and 33, and/or
(iv) a primer set for detecting *Cercospora beticola,* comprising one primer each from the six groups selected from
(iv1) SEQ ID NO: 34 and 35,
(iv2) SEQ ID NO: 36 and 37,
(iv3) SEQ ID NO: 38 and 39,
(iv4) SEQ ID NO: 40 and 41,
(iv5) SEQ ID NO: 42 and 43, and/or
(iv6) SEQ ID NO: 44 and 45.

11. The primer set according to claim 10 comprising at least two, three, or all four primer sets of (i), (ii), (iii), or (iv) in combination with each other.

12. The primer set according to claim 10 or 11, wherein the primer set for detecting BChV comprises the set of sequences as defined by SEQ ID NO: 2, 4, 6, 8, 11 and 13, and/or the primer set for detecting BMYV comprises the set of sequences as defined by SEQ ID NO: 14, 15, 17, 18, 20 and 21, and/or the primer set for detecting BYV comprises the set of sequences as defined by SEQ ID NO: 22, 24, 26, 28, 30 and 32, and/or wherein primer set for detecting *Cercospora beticola* comprises the set of sequences as defined by SEQ ID NO: 35, 37, 39, 41, 43 and 45.

13. The primer set according to claims 10 to 12, wherein at least one primer, preferably at least one forward inner or at least one reverse primer, comprises at least one label, preferably a 5'-label, preferably wherein the label is selected from the group of biotin, FITC and FAM.

14. A use of the system according to claim 8 or 9, for performing the loop mediated isothermal amplification method according to claims 1 to 7 on the at least one target sequence.

15. The use according to claim 14, wherein the result of the loop mediated isothermal amplification method is used to provide individual plants in a field identified as positive for at least one pathogen with a suitable individualized treatment.
